# EUROPEAN PATENT SPECIFICATION

(11) **EP 4 324 418 B1**
(45) Date of publication and mention of the grant of the patent: **11.03.2026**
(21) Application number: 22191101.9
(22) Date of filing: 18.08.2022
(51) Int. Cl.: A61B 18/18

(54) **A COAXIAL CABLE FOR A MICROWAVE ABLATION PROBE**
KOAXIALKABEL FÜR EINE MIKROWELLENABLATIONSSONDE
CÂBLE COAXIAL POUR UNE SONDE D'ABLATION À MICRO-ONDES

(43) Date of publication of application: 21.02.2024
(73) Proprietor: Endowave Ltd., D02 DK18 Dublin 2 (IE)
(72) Inventor: RUVIO, Giuseppe, Dublin 2, D02 DK18 (IE); EATON-EVANS, Jimmy, Dublin 2, D02 DK18 (IE); BOUCHIER-HAYES, Jonathan, Dublin 2, D02 DK18 (IE)
(74) Representative: Barker Brettell LLP

(56) References cited:
- EP-A2- 0 375 840
- JP-A- 2015 080 010
- JP-A- 2021 058 021
- JP-A- H0 669 720
- US-A- 3 230 957
- US-A- 3 947 834
- US-A- 5 705 967
- US-A1- 2012 298 397
- US-A1- 2021 068 897

## Description

### Introduction

This application relates to a coaxial cable. In particular, a coaxial cable for a microwave ablation probe having an operating frequency at which microwave energy is delivered to target tissue. The present application further relates to a microwave ablation system comprising a coaxial cable, and to a method of making a coaxial cable for an ablation probe.

### Technical Background

Thermal ablation can be used to destroy tissue growths within the body which can be malignant. Current ablation systems use applicators that deliver Radio Frequency RF energy or microwave energy to the tissue surrounding an applicator tip. This causes localised heating and destruction of the malignant cells.

Known ablation probes comprise an active tip or applicator generally made of ceramic material which is coupled to a coaxial feed cable arranged to supply electromagnetic energy to the active tip. Typically, coaxial feed cables are semi-rigid structures having excellent electrical performance, with most notably low losses, to ensure the power emitted by the applicator is not attenuated. However, the inventors have identified problems with these semi-rigid structures of coaxial cables, including: a lack of flexibility; and a lack of elasticity.

An endoscopic or other similar device may be used to deliver a microwave ablation probe into the airways of a patient to provide microwave radiation at an intended target lesion within the lungs. The inventors have found that known coaxial feed cables result in a loss of the desired trajectory when an ablation probe is delivered through a torturous path and exits a working channel to reach the target lesion. The loss of the desired trajectory is due to the outer conductor, typically copper, lacking the required elasticity so that when it is deformed, such as bending the coaxial feed cable to manoeuvre a sharp bend, it does not return to its original shape. The desired trajectory can be to maintain a straight path once passing a sharp bend and exiting the working channel. However, due to the outer conductor material properties, the curved path is instead likely to be maintained. As a result of the loss of the desired trajectory, a clinician is unable to accurately position the microwave ablation probe at the position of the target lesion. Moreover, the overall lack of flexibility of the coaxial cable may make it difficult to pass the ablation probe along a long tortuous path without needing to apply excessive force.

It is known to provide a pattern of cuts on a rigid or semi-rigid material in order to provide a greater degree of flexibility and elasticity when a force is being applied. If this technique is applied to an electrical cable however, the removal of material from the conductor may result in a loss of electrical performance.

It is an object of the present application to provide a coaxial cable for a microwave ablation probe that is suitable for use with an endoscopic type of delivery device along a tortuous path without compromising microwave signal strength out of the applicator.

US2021068897A1 discloses a flexible instrument comprising an antenna having a distal tip portion, a proximal base, and an antenna body therebetween. The antenna body comprises a patterned cylindrical structure having a proximal end coupled to the proximal base and a distal end coupled to the distal tip portion. The flexible instrument is configured to generate a radiation pattern from the antenna to ablate tissue.

The applicant is also aware of: US3230957A, which discloses a high frequency therapeutic apparatus; US5705967A, which discloses a high frequency radiating line; JP2015080010A, which discloses an antenna and diversity communication system; JP2021058021A, which discloses a cable and manufacturing method thereof; JPH0669720A, which discloses a leaking co-axial cable; US3947834A, which discloses a Doppler perimeter intrusion alarm system using a leaky waveguide; EP0375840A2, which discloses an apparatus for transmitting high frequency signals; and US2012298397A1, which discloses a leaky coaxial cable.

### Summary of the Invention

The present application provides a microwave ablation system according to the appended claims.

The plurality of cut-outs may each have a length less than half a wavelength of the electromagnetic energy corresponding to the operating frequency of the ablation probe (in use) in the medium within the cut-outs. This may allow the electrical properties of the outer conductor to be maintained and limit losses of signal.

The plurality of cut-outs may each have a length within a range of between 0.5 mm to 12 mm, or more preferably between 1 mm to 8 mm. The plurality of cut-outs may each preferably have a length less than 4 mm. This may limit the loss of signal when a microwave signal is carried by the coaxial cable.

By providing a plurality of cut-outs in the outer conductor of the coaxial cable having a certain size a microwave ablation probe that is suitable for use with an endoscopic type of device along a tortuous path without compromising microwave signal strength out of the applicator is provided. The cut-outs on the outer conductor of the coaxial cable allow for the desired flexibility and elasticity of the coaxial cable required to provide a trajectory that can maintain a straight path once passing a sharp bend. The cut-outs also aid flexibility and facilitate passage along a long tortuous path. By using cut-outs each with a size determined according to the microwave signal the electrical properties of a complete outer conductor without any cut-outs is retained. This allows the microwave signal to propagate along the length of the coaxial cable without significant power-leakage or losses through the cut-outs, while also providing the improved elasticity and flexibility of the cable.

The plurality of cut-outs may each extend along a helical path around the outer conductor. Each cut-out may therefore extend around a longitudinal axis of the coaxial cable. The helical shape of the cut-outs may allow the coaxial cable to have the same degree of flexibility in all directions.

Each of the plurality of cut-outs may extend along part of a combined continuous helical path along the length of the coaxial cable. The plurality of cut-outs may be spaced apart along the continuous helical path to form an intermittent helical cut-pattern. This may allow the cut-outs to lie on a single helical path along the cable to give even flexibility, while having a size suitable to limit the losses of the microwave signal.

The plurality of cut-outs may each extend only partly around the outer conductor around an axis of the coaxial cable, preferably in a plane normal to the longitudinal axis, each spaced by a distance along the length of the coaxial cable.

Each of the plurality of cut-outs may be offset around the longitudinal axis of the coaxial cable relative to an adjacent cut-out. The plurality of cut-outs may therefore form a staggered cut-pattern.

The plurality of cut-outs may be grouped together. The groups may be spaced apart along the length of the coaxial cable. Each consecutive cut-out in a group may be offset around the longitudinal axis of the coaxial cable in relation to an adjacent cut-out in that group of cut-outs.

Each of the plurality of cut-outs may be offset relative to adjacent cut-outs along the length of the coaxial cable such that the plurality of cut-outs are arranged in a brick pattern. The plurality of cut-outs may be arranged such that gaps between cut-outs, or gaps between the ends of a cut-out, around the longitudinal axis of the coaxial cable, do not overlap with corresponding gaps of adjacent cut-outs along the length of the coaxial cable.

The plurality of cut-outs may each extend completely through the thickness of the outer conductor.

The plurality of cut-outs may only extend through the outer conductor, and not extend into the dielectric. This may help maintain the electrical properties of the cable.

The operating frequency may be a microwave frequency. The operating frequency may be in a range between 10.0 GHz and 0.5 GHz. The operating frequency may be in a range of 2.4GHz to 2.5GHz, and is preferably 2.45GHz. This may be suitable for providing microwave ablation therapy.

The plurality of cut-outs may be arranged to form a first portion of the coaxial cable having a first degree of flexibility and a second portion of the flexible cable having a second degree of flexibility greater than the first. This may allow the flexibility of the cable to be tailored along different parts of its length. The plurality of cut-outs in the first portion may have a lower number density compared to those of the second portion.

The feeding cable may comprise or contain a propagating medium within the cut-outs. The propagating medium may be a material arranged to fill each of the cut-outs, or a fluid (e.g. a gas such as air of a fluid coolant such as water) or vacuum that fills each of the cut-outs. The length of the cut-outs may be less than half the wavelength of electromagnetic energy having a frequency of the ablation probe operating frequency in the propagating medium.

The feeding cable may further comprise a sheath around the coaxial cable. The sheath may be arranged to form a water-resistant barrier around the coaxial cable. The sheath may be formed from: a dip coating of the coaxial cable, a shrink-fit polymer tube around the coaxial cable, or an extruded jacket over the coaxial cable.

The sheath may comprise a metalized layer around the coaxial cable.

The feeding cable may further comprise one or more conducting wires arranged between the outer surface of the coaxial cable and the sheath. The conducting wires may extend along the length of the coaxial cable. The conducting wires may electrically connect at least some of the plurality of cut-outs.

The one or more conducting wires may each extend longitudinally along the length of the coaxial cable. The one or more conducting wires may form a conducting braid or coil around the feeding cable.

The present application further provides a method of making the coaxial cable of the ablation system of the first aspect, the method being as defined in the appended claims.

Cutting the plurality of cuts-outs may comprise controlling the laser beam by tuning the laser beam to remove material from the outer conductor but not from the dielectric.

Except where mutually exclusive, a feature described in relation to any one of the above aspects may be applied to any other aspect.

### Description of the Figures

Embodiments of the invention will now be described, by way of example only, with reference to the accompanying drawings, in which:
**Figure 1** shows a schematic diagram of a microwave ablation system comprising a feeding cable, with an exploded view of a segment of the feeding cable showing a plurality of cut-outs in an outer conductor of a coaxial cable forming part of the feeding cable;
**Figure 2** shows a close-up of the feeding cable of Figure 1;
**Figure 3a** shows a first cross-sectional view in a plane normal to the longitudinal axis of the coaxial cable of Figure 1;
**Figure 3b** shows a second cross-sectional view in a plane normal to the longitudinal axis of the coaxial cable of Figure 1;
**Figure 4A** shows a perspective view of a coaxial cable having cut-outs arranged in a brick cut-pattern;
**Figure 4B** shows a close-up of the coaxial cable of Figure 4A;
**Figure 4C** shows a side-view of a length of the coaxial cable of Figure 4A;
**Figure 5A** shows a perspective view of a coaxial cable having cut-outs arranged in a staggered cut-pattern;
**Figure 5B** shows a close-up of the coaxial cable of Figure 5A;
**Figure 5C** shows a side-view of a length of the coaxial cable of Figure 5A;
**Figure 6A** shows perspective view of a coaxial cable having cut-outs arranged in an intermittent helical cut-pattern;
**Figure 6B** shows a close-up of the coaxial cable of Figure 6A;
**Figure 6C** shows a side-view of a length of the coaxial cable of Figure 6A;
**Figure 7A** shows a perspective view of a coaxial cable having portions of differing flexibility;
**Figure 7B** shows a side-view of the coaxial cable of Figure 7A;
**Figure 8A** shows a side view of another embodiment of a coaxial cable;
**Figures 8B** and **8C** show cross sectional views through the coaxial cable of Figure 8A; and
**Figure 9** shows a method of making a coaxial cable.

### Description

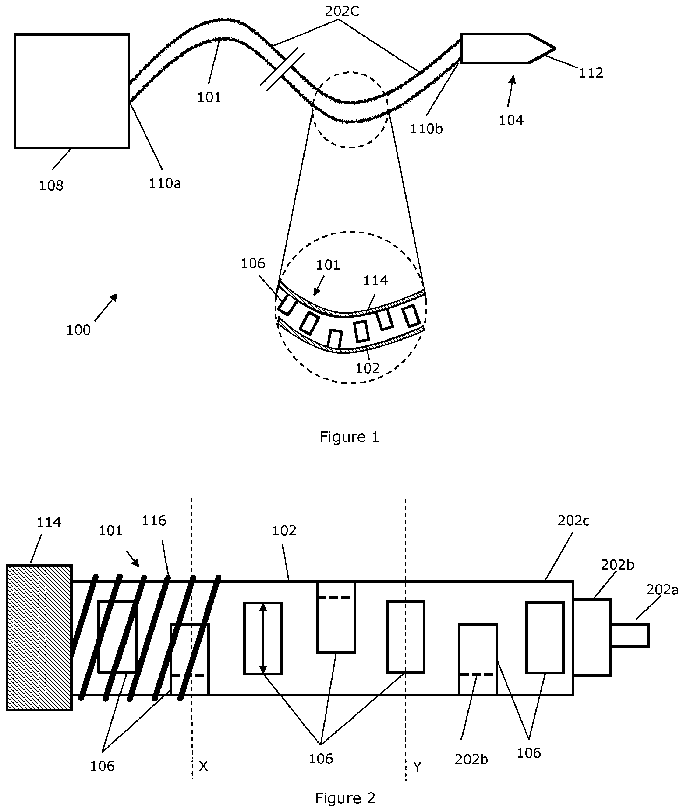

A microwave ablation system 100 is shown diagrammatically in Figure 1. The system 100 comprises: a feeding cable (or feed cable) 101, an ablation probe 104, and a signal generator 108. The feeding cable 101 comprises a coaxial cable 102, as seen in the exploded portion view of Figure 1. The ablation probe 104 comprises an applicator 112, arranged at a distal end of the ablation probe 104. The applicator 112 is arranged to emit microwave radiation to a target location in order to ablate tissue. The signal generator 108 is arranged to generate microwave signals which propagate along the length of the coaxial cable 102, such that the microwave energy is delivered to target tissue, via the applicator 112 of the ablation probe 104.

The coaxial cable 102 is arranged to supply electromagnetic energy, from the signal generator 108, to the applicator 112 of the microwave ablation probe 104. The applicator may be a ceramic tip arranged to emit microwaves. The coaxial cable 102 is coupled at a first (or proximal) end 110a to the frequency signal generator 108, in order to receive an electromagnetic signal, and is further coupled at a second (or distal) end 110b to the microwave ablation probe 104, in order to provide the microwave signal to the applicator 112 of the microwave ablation probe 104. In this application, the terms "distal" and "proximal" are taken relative to the treatment site when the ablation probe is positioned for use - the distal end of the feeding cable 101 or coaxial cable 102 is that closest to the treatment site and the proximal end is that furthest from the treatment site (and closest to the signal generator 108). A control device (not shown in the Figures) such as a handle may be provided towards the proximal end of the feeding cable 101 so that it can be manipulated and positioned by the user.

The coaxial cable 102 is of a length suitable to extend from the frequency signal generator 108 to the microwave ablation probe 104 at a desired treatment site. The coaxial cable 102 may be provided within or form part of the feeding cable 101. The feeding cable 101 is connected between the frequency signal generator 108 and the microwave ablation probe 104. Only part of the length of the feeding cable 101 is shown in Figure 1, and the components shown are not drawn to scale to aid illustration.

The ablation probe 104 and feeding cable 101 of the present disclosure may be suitable for insertion into the body to reach the desired treatment site, such as a malignant tissue growth. In order to reach the desired treatment site, the ablation probe 104 and feeding cable 101 may be suitable for insertion through the working channel of an internal anatomy access device. By internal anatomy access device we mean any device which may be placed within the anatomy of a patient, the device having a working channel for insertion of instruments to the desired target location within the body. The internal anatomy device may be an intraluminal delivery device arranged to be delivered along an anatomical lumen of the patient e.g. the trachea and the pathways of the bronchi in the lungs or the oesophagus. The ablation probe 104 may, for example, be used endoscopically or using an ENB system in order to reach a variety of disease locations within the body. The ablation probe 104 and feeding cable may therefore have an overall flexibility such that they can be inserted through the working channel of the endoscope. In other embodiments, the ablation probe 104 and feeding cable 101 may be used with other types of intraluminal delivery device such as specific types of endoscope e.g. a bronchoscope or a navigation system such as a lung navigation system e.g. an ENB system.

In the presently described embodiment, a single coaxial cable is provided that runs from a proximal end of the feeding cable connected to the signal generator to a distal end of the feeding cable connected to the ablation probe. In other embodiments, the coaxial cable may extend only part way along the length of the feeding cable (with other types of connection also provided), or several different interconnected cables may be used along the length of the feeding cable.

The signal generator 108 is arranged to produce an oscillating signal at a frequency suitable for locally heating tissue at the ablation site (i.e. at the operating frequency of the ablation prove). The signal generator 108 provides a signal having a frequency within the Radio Frequency (RF) sub-section of the electromagnetic spectrum, more specifically a microwave signal. In some embodiments, the operating frequency of the ablation probe may be within a range of 10.0 GHz to 0.5 GHz. In some embodiments, the operating frequency of the ablation probe may be within a range of 1.0 to 4.0 GHz, or more preferably 2.4 GHz to 2.5 GHz. In particular, at a frequency of 2.45 GHz. Using an operating frequency within these ranges allows for the required microwave treatment at the treatment site. The ranges of operating frequency given in this paragraph are preferred examples only, and the operational frequency may vary in other embodiments.

A close-up of the feeding cable 101 is shown in Figure 2. The coaxial cable 102 comprises an inner conductor 202a, a dielectric 202b, and an outer conductor 202c. The dielectric 202b is situated between the inner conductor 202a and the outer conductor 202c. The outer conductor may be formed from a tube of electrically conducting material, such as a metal tube (e.g. copper). The dielectric may be any suitable dielectric, such as PTFE. The inner conductor may be any suitable conducting wire, such as silver plated copper wire. The feeding cable 101 may further comprise additional components, such as, an insulative sheath 114. The insulative sheath 114 may fit around the outer surface of the outer conductor 202c and may form an outer part or surface of the feed cable 101. The insulative sheath may form a close fit around the outer conductor 202c of the coaxial cable 102. The insulative sheath may be arranged to form a water-resistant barrier around the coaxial cable 102, and may prevent fluid ingress into the coaxial cable 102. The sheath 114 may be formed by dip coating the coaxial cable in a water-resistant material, shrink-fitting a polymer tube around the coaxial cable 102, or extruding a jacket over the coaxial cable in a continuous manufacturing process. Figure 2 shows a cut-away view in which the layers of the coaxial cable, conducting wire 116 (described later) and the surrounding insulative sheath are cut-away at successive points along the cable so that each of the layers are visible. It will be understood that these layers may extend along all or part of the length of the feeding cable.

In other embodiments, the feeding cable 101 (and/or the coaxial cable 102) may comprise additional or alternative components. For example, the insulative sheath 114 may not be provided. In some embodiments, the feeding cable 101 may comprise one or more coolant flow channels extending along its length. These may provide a flow of coolant to the applicator 112. In some embodiments, two or more coolant flow channels may be provided, one carrying a flow of coolant towards the applicator, the other a flow of coolant away from the applicator. The coolant may be water, and may be provided from a coolant circulation system not shown in the figures. In some embodiments, one or more of the coolant channels may be formed within the feeding cable in a space defined between the outer conductor 202c of the coaxial cable 101 and a tube surrounding the coaxial cable 102. The inside wall of the tube may be spaced apart from the outer surface of the coaxial cable to form a coolant channel between them. The coolant channel may be further divided into separate channels to form a coolant circuit.

The coaxial cable 102 comprises a plurality of cut-outs 106. More specifically, the outer conductor 202c comprises the plurality of cut-outs 106. Each cut-out is formed by a hole or aperture in the outer conductor 202c. The cut-outs may be formed by removing material from the outer conductor as will be described in more detail later. The cut-outs may be formed using any suitable means that can provide a hole in the outer conductor 202d. The plurality of cut-outs 106 each extend completely through the thickness of the outer conductor to form weak points in the structure of the outer conductor to modify its mechanical properties.

The cut-outs each have a length (i.e. maximum or dominant size) that is less than 10% of the wavelength in free-space at the operating frequency of the ablation probe. In other embodiments, the cut-outs may each have a length (i.e. maximum or dominant size) that is less than 2% of the wavelength in free-space at the operating frequency of the ablation probe In particular, the plurality of cut-outs 106 each have a length which is less than half a wavelength of the electromagnetic energy, in the medium within the cut-outs, at the operating frequency of the ablation probe 104 (when the ablation probe is in use).

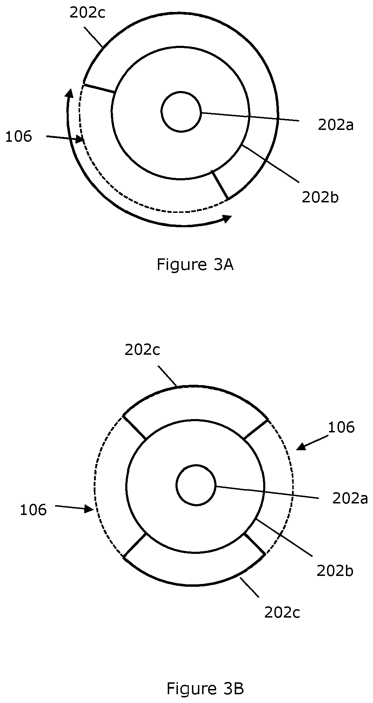

By length of the cut-outs we mean the dominant size. In other words, the largest length of a side or edge of a cut-out extending along or around the outer conductor. For example, where the cut-outs are rectangular in shape as shown in Figure 2, the size of the cut-out is the length of its longest side as shown by the arrows in Figure 2 and 3A. More generally, where a cut-out has sides that are not straight (e.g. the cut-out is elliptical in shape), the length of a cut-out is the longest or major axis of the shape of that cut-out.

By tailoring the size and shape of the cut-outs in this way, the inventors have found that the mechanical requirements of the cable can be satisfied (e.g. a suitable level of flexibility and/or elasticity provided), without causing excess power-leakage through the outer-conductor that would otherwise impact on the ablation provided by the applicator 112. By providing cut-outs 106 within the outer conductor 202c its mechanical properties can be tailored for insertion into a delivery device inside the patient. For example, by providing points of weakness in the outer conductor its flexibility and elasticity can be increased. By increasing the flexibility of the outer conductor 202c it may be easier for the user to inert the coaxial cable 102 (and the part of the feeding cable 102 in which it is located) along a tortuous path through a delivery device within the anatomy of the patent. Additionally or alternatively, by increasing the elasticity of the coaxial cable 202c it may more readily return to its original shape when it leaves the end of a delivery device. This may otherwise cause a loss of trajectory caused by the feeding cable retaining its curved shape.

The use of cut-outs in the outer conductor 202c can be particularly advantageous where the outer conductor 202c is made from an otherwise continuous tube or sheath of material e.g. a metal such as copper. Such an outer conductor may be particularly inflexible and have a low elasticity, meaning that it has a "shape memory" causing it to be difficult to direct once outside of a delivery device.

In some embodiments, the cut-outs 106 may each have a length which is less than half a wavelength in air of the electromagnetic energy corresponding to the operating frequency. This may be suitable for a number of designs of feeding cable in which the outer conductor is surrounded by air during use, or in which the medium within the cut-outs is air (or a medium with a similar permittivity). In the presently described embodiment, the cut-outs 106 may be filled with air trapped by the insulative sheath 114.

More generally, the feeding cable 101 may comprise or contain a propagating medium which is disposed within each of the cut-outs 106. The cut-outs 106 may each have a length which is less than half of a wavelength in the propagating medium at the operating frequency of the ablation probe. For example, the feeding cable 101 may comprise a material arranged to fill each of the cut-outs 106, or a layer which extends into the cut-outs 106 (such as the insulating sheath 114). In other examples, the feeding cable 101 may contain a propagating medium such as air (or other gas, or a vacuum) within the cut-outs 106. In other embodiments, the feeding cable may contain a propagating medium such as water within the cuts-outs 106. This may be the case, for example, if a coolant flow channel is defined by the outer surface of the outer conductor 202c, and the cut-outs are filled with water as it flows along the coolant flow channel.

In the described embodiment the cut-outs all contain the same propagating medium, and so are sized and/or shaped according to the same length parameter. This may not be the case in other embodiments, in which some of the cut-outs contain a different propagating medium, and so are sized and/or shaped according to their respective propagating medium.

In some embodiments, the plurality of cut-outs 106 each have a length within a range of between 0.5 mm to 12 mm, or preferably between 1 and 8 mm. In some embodiments, the plurality of cut-outs 106 may each have length less than 4 mm. This corresponds to a size in a range suitable to prevent leakage of radiation at the ablation probe operating frequencies defined elsewhere herein.

The plurality of cut-outs 106 may be arranged along the length of the outer conductor 202c in any suitable pattern or distribution. Other sizes and shapes of cut-outs may also be used other than those shown in the Figures. The distribution, size and shape may be chosen according to the desired resulting mechanical properties of the coaxial cable, while being within the maximum cross-sectional size limit to ensure the desired electric properties.

Each of the plurality of cut-outs 106 are shown in Figure 2 to extend from the outer surface of the outer conductor to the outer surface of the inner dielectric insulator 202b, such that a sector of the area occupied by any one of the plurality of cut-outs 106 is entirely removed. Each of the plurality of cut-outs therefore extend completely through the thickness of the outer conductor. The plurality of cut-outs may only extend through the outer conductor, and do not extend into the dielectric. This helps to reduce to remove any impact on the electrical properties of the coaxial cable.

The extent of the cut-outs through the outer conductor 202d is illustrated in Figures 3a and 3b. A first cross-sectional view in a plane normal to the longitudinal axis of the coaxial cable 10 at a position X is shown in Figure 3a. A second cross-sectional view in a plane normal to the longitudinal axis of the coaxial cable is shown in Figure 3b at a different position (Y) along the length of the coaxial cable 102. Each of the plurality of cut-outs 106 are shown in Figure 3A and Figure 3B to extend from the outer surface of the outer conductor 202c to the outer surface of the dielectric 202b, such that a sector of the cross-sectional area of outer conductor otherwise occupied by any one of the plurality of cut-outs 106 is entirely removed. As can be seen from Figure 3A and Figure 3B, the plurality of cut-outs 106 of each figure are offset in relation to one another. The offset between the plurality of cut-outs 106 reflects how the plurality of cut-outs are arranged along the length of the coaxial cable 102 and around the circumference of the coaxial cable 102.

In the embodiment shown in Figure 2, the feeding cable further comprises a conducting wire 116 which is arranged between the outer surface of the outer conductor 202a and the sheath 114. The conducting wire may be a metallic wire, and is arranged to electrically connect the cut-outs 106 (or at least some of them). This may further reduce the attenuation of the microwave signal along the coaxial cable. In the presently described embodiment, the conducting wire forms a coil around the outer surface of the coaxial cable. In other embodiments, a plurality of conducting wires may be provided, which may be formed into a braid around the outer conductor. In yet other embodiments, one or more conducting wires may extend longitudinally along the length of the outer conductor to electrically connect the cut-outs.

In some embodiments, the conducting wire or wires 116 may form thermocouples arranged to monitor the temperature of the coaxial cable. The thermocouples may be connected to a control unit (not shown) provided at the distal end of the feeding cable. In some embodiments, the conducting wire 116 or wires may not be provided.

Additionally or alternatively to the conducting wire or wires 116, the sheath 114 may comprise a metalized layer around the coaxial cable. This may also help to reduce the attenuation of microwaves along the coaxial cable.

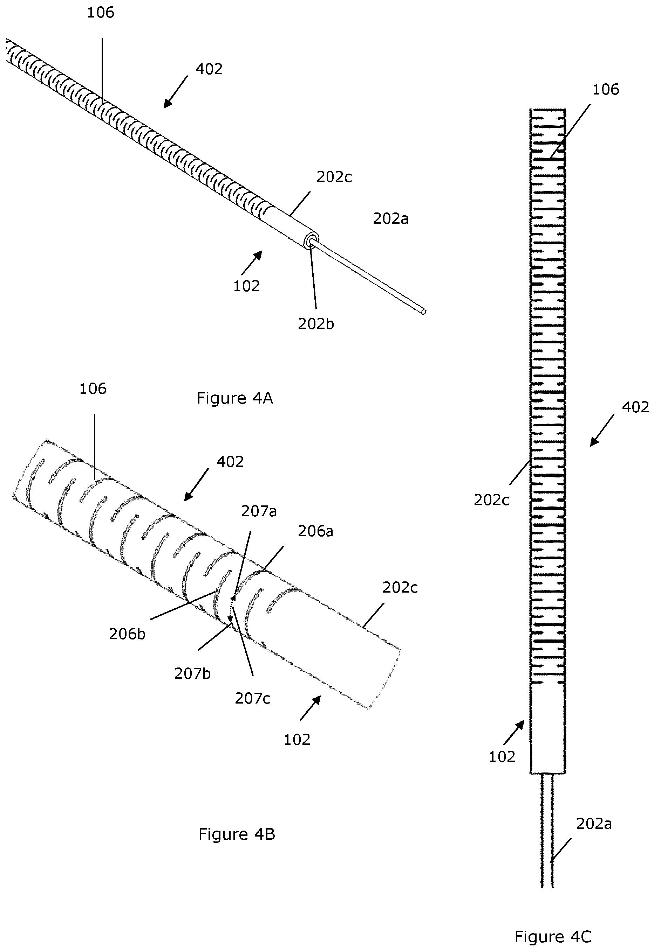

The plurality of cut-outs in the outer conductor may be arranged along the length (or a portion of the length) of the coaxial cable in various different patterns, referred to herein as "cut-patterns". The plurality of cut-outs 106 may take the form of any one of the following cut-patterns; a brick cut-pattern 402, a staggered cut-pattern 502, or an intermittent helical (or spiral) cut-pattern 602, as shown in Figures 4A to 4C, 5A to 5C, and 6A to 6C, respectively. These are provided as preferable examples only, and the plurality of cut-outs 106 may be arranged in other cut-patterns (not shown in the Figures).

Figures 4A to 4C show an embodiment of a coaxial cable having a brick (or bricked) cut-pattern. A close-up of the brick cut-pattern is shown in Figure 4B, and a side-view of a length of the brick cut-pattern is shown in Figure 4C. The brick cut-pattern is formed by a series of cut-outs 106 spaced apart along the length of the coaxial cable 102. In the presently described embodiment, the cut-outs are equally spaced apart along the longitudinal axis of the coaxial cable. In other embodiments, the cut-outs 106 may be unequally distributed (but still form a brick pattern). Each individual cut-out forming the brick pattern extends around a portion of the circumference of the outer conductor 202c, i.e. each cut-out 106 extends part way around the circumference of the outer conductor 202c in a plane normal to its longitudinal axis. Each cut-out 106 of the brick pattern is offset in relation to an adjacent cut-out along the length of the coaxial cable. In other words, the ends 207a, 207b of a first cut-out 206a are offset around the circumference of the outer conductor 202c in relation to the ends of an adjacent second cut-out 206b. Adjacent cut-outs 106 may be positioned around the circumference of the coaxial cable so that the gaps between their ends (one of which is labelled 207c in the Figures) do not overlap with gaps of adjacent cut-outs along the length of the cable so that a brick pattern is formed. In the embodiment shown in the Figures, the cut-outs 106 alternative between cuts-outs having the same orientation about the circumference of the outer-conductor as the first cut-out 206a and those have the same orientation of the second cut-out 206b. In the present embodiment, each of the cut-outs 106 has the same length about the outer conductor 202c.

In other embodiments, other variations of the brick pattern may be provided. For example, the cut-outs may be arranged in rows of cut-outs, with cut-outs of each row extending in the same normal plane to the longitudinal axis of the coaxial cable. Each row may comprise two or more cut-outs and two or more corresponding gaps between them. The cut-outs of each row are offset around the circumference of the coaxial cable compared to those of adjacent rows so that the gaps between cut-outs of one row do not overlap with the gaps between cut-outs of an adjacent row to form a brick pattern as described above.

In yet other embodiments, the cut-outs forming the brick pattern may be angled relative to a plane normal to the longitudinal axis of the cable.

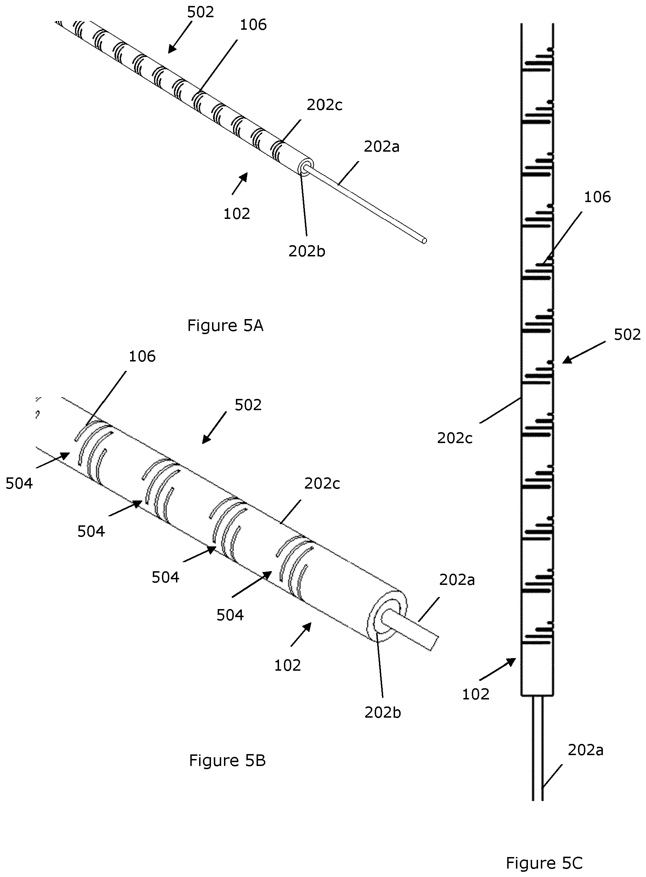

Figures 5A to 5C show an embodiment of a coaxial cable having a staggered cut-pattern. A close-up of the staggered cut pattern is shown in Figure 5B, and a side-view of a length of the staggered cut-pattern is shown in Figure 5C. Each of the cut-outs forming the staggered cut-pattern extend only partly around the circumference of the outer conductor 202c around the longitudinal axis of the coaxial cable 102. As shown in the Figures, they may each extend in a plane normal to the longitudinal axis. In other embodiments, the cut-outs forming the staggered cut-pattern pattern may be angled relative to a plane normal to the longitudinal axis of the cable. The cut-outs 106 forming the staggered pattern are each offset around the circumference of the outer conductor 202c in relation to an adjacent cut-out along the length of the coaxial cable 102. The cut-outs are offset such that a start and end of each cut-out does not have the same position around the longitudinal axis of the coaxial cable compared to the start and end point of an adjacent cut-out.

The staggered cut pattern may preferably comprise groups 504 of two of more cut-outs 106 in each group. In the described embodiment, each group comprises four staggered cut-outs 106. The cut-outs of each group may be equally spaced apart from each other, and may be of equal length around the circumference of the outer conductor. In other embodiments the cut-outs in each group may be unequally spaced and/or of different lengths/sizes. In other embodiments, the staggered cut-outs may not be grouped.

In the presently described embodiment, the groups 504 are equally spaced apart along the longitudinal axis of the coaxial cable. In other embodiments, the groups may be unequally spaced. This may allow the mechanical properties of the cable to be varied along its length.

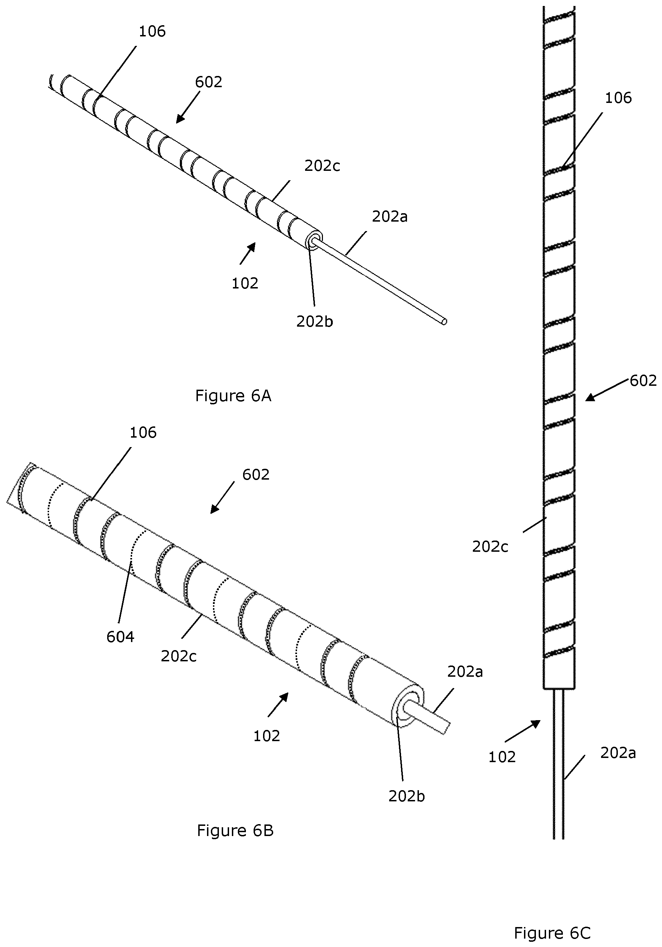

An embodiment in which the plurality of cut-outs are arranged in an intermittent helical (or spiral) cut-pattern is shown in Figures 6A to 6C. A close-up of the intermittent helical cut-pattern is shown in Figure 6B, a side-view of a length of the intermittent helical cut-pattern is shown in Figure 6C. The intermittent helical cut pattern comprises a plurality of cut-outs 106 where each individual cut-out extends helically along a portion of the surface of the outer conductor 202c. The cut-outs 106 forming the intermittent helical pattern all extend along part of a path defining one combined continuous helical path (shown by the broken line labelled 604 in Figure 6B) along the length of the coaxial cable 102. The cut-outs 106 are spaced apart along the continuous combined helical path so that gaps along the path are formed. The intermittent helical cut-pattern may therefore be referred to as an "interrupted" helical cut-pattern. In the described embodiment, each of the cut-outs 106 forming the intermitted helical cut-pattern may be evenly distributed over the combined helical path (e.g. they may have the same length and the gaps between them may be the same). In other embodiments, the cut-outs 106 may be of unequal length and/or spacing. In the described embodiment, each of the plurality of cut-outs extend more than a single turn about the longitudinal axis of the coaxial cable. In other embodiments, the cut-outs 106 may extend less than a complete turn around the longitudinal axis. The intermitted helical cut pattern may be advantageous in providing an equal flexibility of the coaxial cable in all directions. In the present embodiment, the pitch of the continuous helical path 604 is constant along the length of the coaxial cable. In other embodiments, the pitch may be varied along the length of the cable. This may allow regions of different flexibility.

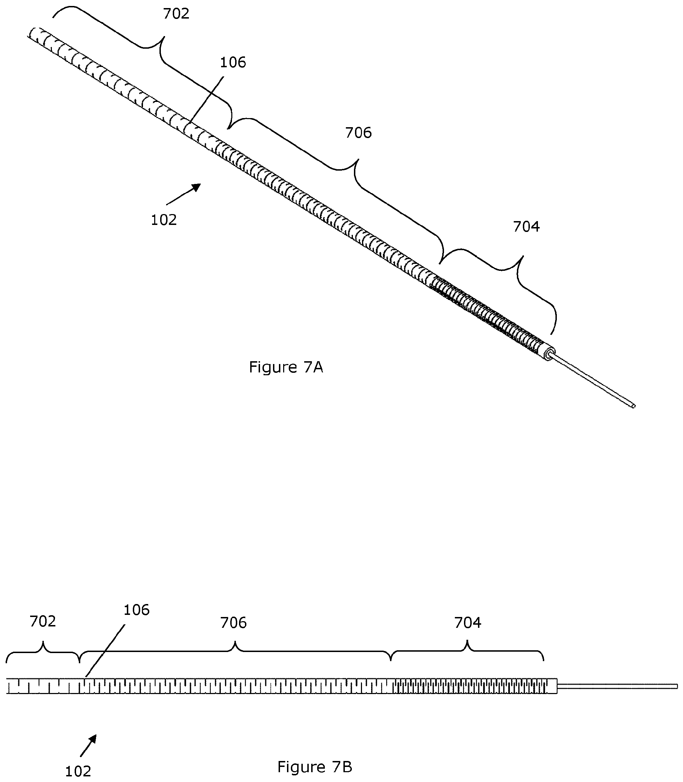

In some embodiments, the coaxial cable 102 may comprise two or more portions having differing degrees of flexibility. For example, as illustrated in Figures 7A and 7B, the coaxial cable 102 may comprise a first region 702 (or portion) having a first flexibility, a second region 704 having a second flexibility (different from the first) and a third region 706 having a third flexibility (different from the first and second). Differing arrangements of the plurality of cut-outs 106 along the coaxial cable 102 may allow for different degrees of flexibility in each of the first, second and third regions along the length of the coaxial cable 102. The differing degrees of flexibility in each region may be desirable as, for example, in the first region 702 of the coaxial cable 102 near the first end 110a of the coaxial cable 102 that is coupled to the frequency signal generator 108, a relative low flexibility may be provided. This may allow the cable to be kept in a more rigid state, thus preventing unnecessary bending or curving of the coaxial cable 102. This may make it easier to push the feeding cable through the working channel of a delivery device. In the second region 704 of the coaxial cable 102 near (or closer to) the second end 110b coupled to the microwave ablation probe 104, the plurality of cut-outs 104 may be arranged to provide a relative higher degree of flexibility compared to in the first region. This may be preferred as this will allow the feeding cable 101 to more easily follow a curved or tortuous path near the desired treatment site. The second region 104 may also have a greater elasticity compared to the first region 702 so that the coaxial cable continues on the desired path after leaving a delivery device. The third region 706 of the presently described embodiment is arranged in between the first region 702 and the second region 704. The plurality of cut-outs 106 in the third region 706 may be arranged to provide a greater degree of flexibility than the first region 702 and a greater degree of rigidity (i.e. lower flexibility) than the second region 704. This may allow for a more gradual transition of rigidity from the first region 702 to flexibility at the second region 706 along the length of the coaxial cable 102.

In order to provide a different flexibility the cut-outs 106 within the first, second and third regions 702, 704, 706 may have different shapes, sizes or separation to those of the other regions. In the described embodiment, the density of cut-outs 106 varies between each portion 702, 704, 706 of the coaxial cable, with the first portion 702 having a first density, the second portion 704 a second density and the third portion a third density. The first density is less than the second density, and the third density is between the first and second density. By density we mean the number of cut-outs per unit length of the coaxial cable (i.e. the number density of the cut-outs). In other words, the plurality of cut-outs in the first portion 702 have a greater separation between each of them to those of the third portion 706, which have a greater separation compared to those of the second portion 704. In these embodiments, the size and shape of each of the cut-outs may be the same (or the size and shape may vary according to a uniform pattern along the length of the coaxial cable).

Additionally or alternatively, in other embodiments, the flexibility may be varied between the different portions of the coaxial cable by changing the relative size and/or shape of the cut-outs. For example, corresponding cut-outs of the first portion may have a smaller cross-sectional size to those of the third portion 706, and a smaller cross-sectional size compared to those of the second portion 704 (within the maximum size determined by the desired electrical properties of the cable described above).

Although three regions of the coaxial cable having different degrees of flexibility are shown in Figures 7A and 7B more generally there may be at least two regions in which the plurality of cut-outs are arranged to provide a different degree of flexibility. For example, the third portion 706 may be omitted in some embodiments, or additional portions with different degrees of flexibility may be provided. Moreover, the portions 702, 704, 706 may in some embodiments extend over only part of the coaxial cable, rather than its entire length as shown in the Figures. The first portion 702 may generally be closer to the proximal end of the cable compared to the second portion 704.

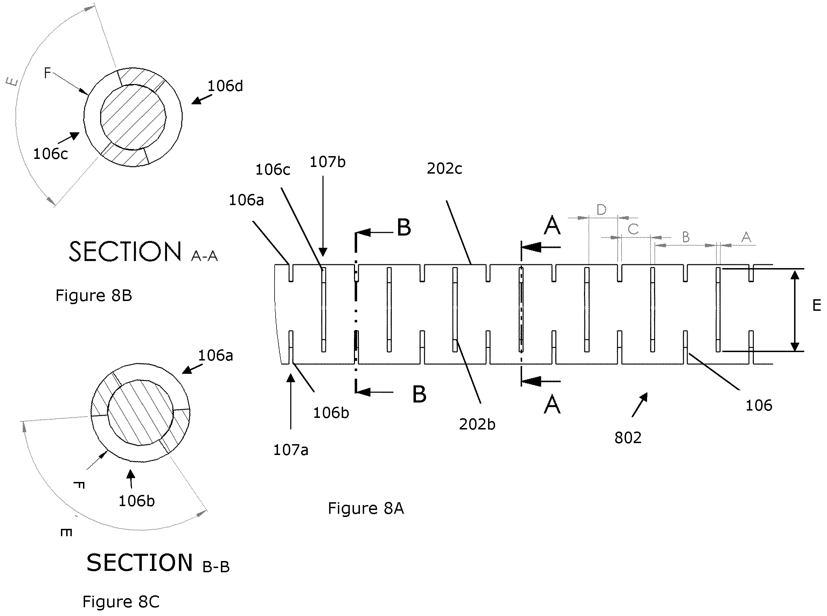

Figures 8a to 8c show a side view and two cross-sectional views of a coaxial cable 802 according to one embodiment of the present application. In this embodiment, the plurality of cut-outs 106 are arranged in a brick pattern as described above. In this example of the brick pattern, each row of the plurality of cuts-outs 106 comprises two cut-outs. As can be seen in the Figures, a first row 107a comprises cut-outs 106a and 106b, which each extend only partly around the circumference of the outer conductor 202c, and are spaced apart around the circumference to form gaps between them. A second row 107b comprises cut-outs 106c and 106d, which again each extend only partly around the circumference of the outer conductor 202c, and are spaced apart around the circumference to form a gaps between them. The cut-outs of the first row are offset around the circumference of the outer conductor 202c so that the gaps between the cut-outs of adjacent rows do not overlap. The cut-outs 106 of the first row are in a first orientation, with the cut-outs of the second row being in a different, second orientation. The brick pattern is formed by further rows alternating between cut-outs having the same orientation as those of the first row, and those having the same orientation of the second row.

In the embodiment shown in Figures 8a-c the size and arrangement of the cut-outs may be characterized by the following parameters labelled in the Figures:
i) "Cut-out width" A, defined as the cross-sectional size of the cut-out measured in a direction along the length of the coaxial cable. The cut-out width is 1‰ (i.e. 0.1%) of the operating wavelength in free-space in the present embodiment, but may be in a range between 0.6‰ to 5‰ (i.e. 0.06% to 0.5%) of the operating wavelength in free-space.
ii) "Cut-out pattern spacing" B, defined as the distance along the length of the coaxial cable between cut-outs of rows having the same orientation (e.g. between cut-outs of rows having the same orientation as the second row as shown in the Figure 8a). The cut-out pattern spacing B in the present embodiment is 8‰ (i.e. 0.8%) of the operating wavelength in free-space, but may be 3‰ to 8% (i.e. 0.3% to 8%) of the operating wavelength in free-space.
iii) "First cut-out row spacing" C, defined as the distance along the length of the coaxial cable between adjacent rows of cut-outs that is between rows having the first orientation of cut-outs, and rows having the second orientation of cut-outs. The First cut-out row spacing C in the present embodiment is 4‰ (0.4%) of the operating wavelength in free-space , but may be 3‰ to 4% (i.e. 0.3% to 4%) of the operating wavelength in free-space.
iv) "Second cut-out row spacing" D, defined as the distance along the length of the coaxial cable between adjacent rows of cut-outs that is between rows having the second orientation of cut-outs, and rows having the first orientation of cut-outs. The second cut-out row spacing D in the present embodiment is 4‰ (i.e. 0.4%) of the operating wavelength in free-space, but may be 3‰ to 4% (i.e. 0.3% to 4%) of the operating wavelength in free-space.

In this example, the width of the coaxial cable is 6‰ (i.e. 0.6%) of the wavelength in free-space, and may be in the range 2‰ to 16‰ (0.2% to 1.6%) of the wavelength in free-space. The length of the cut-outs (marked E in the Figurers) is 15‰ (i.e. 1.5%) of the operating wavelength in free-space, and may be in the range 3‰ to 10% (i.e. 0.3% to, but not including, 10%) of the operating wavelength in free-space.

The parameters given above have been found to provide a preferred balance of the mechanical and electrical properties of the coaxial cable. They are however to be understood as only one illustrative preferred example. The operating wavelength refers to the wavelength in free space at the operating frequency of the ablation probe defined above.

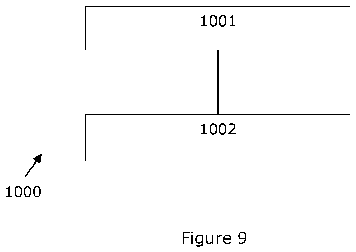

A method 1000 of making a coaxial cable 102 of the present application is shown in a block diagram in Figure 9. The method 1000 comprises a Step 1001, which comprises providing a coaxial cable having: an inner conductor, an outer conductor; and a dielectric situated between the inner conductor and the outer conductor. The method 1000 further comprises a Step 1002 which comprises cutting a plurality of cut-outs in the outer conductor using a laser beam to remove material from the outer conductor. The inventors have found that laser cutting is a particularly advantageous method of producing cut-outs in the outer conductor because cut-outs that are small in size can be accurately manufactured. In Step 1002, cutting the plurality of cuts-outs may further comprise controlling the laser beam by tuning the laser beam to remove material from the outer conductor, but not from the dielectric. This allows the laser cutting to remove only material from the outer conductor, and not the dielectric.

The present application is not limited to laser cutting being used to form the cut-outs, with other methods such as milling or grinding being used in other embodiments.

Various modifications will be apparent to the skilled person without departing form the scope of the claims. Any feature disclosed in connection with one embodiment may be used in combination with the features of another embodiment.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination. The applicant hereby gives notice that new claims may be formulated to such features and/or combinations of such features during the prosecution of the present application or of any further application derived therefrom.

For the sake of completeness, it is also stated that the term "comprising" does not exclude other elements or steps, the term "a" or "an" does not exclude a plurality, a single processor or other unit may fulfil the functions of several means recited in the claims and any reference signs in the claims shall not be construed as limiting the scope of the claims.

## Claims

1. A microwave ablation system (100), comprising:
a signal generator (108), arranged to generate a signal at an operating frequency at which microwave energy is delivered to target tissue;
a microwave ablation probe (104) comprising an applicator (112) arranged to emit microwave radiation to a target location; and
a feeding cable (101) arranged to connect the applicator (112) to the signal generator (108), the feeding cable (101) comprising a coaxial cable (102) connected between the signal generator (108) and the applicator (112), the coaxial cable (102) comprising:
an inner conductor (202a);
an outer conductor (202c); and
a dielectric (202b) arranged between the inner conductor (202a) and the outer conductor (202c),
wherein the outer conductor (202c) comprises a plurality of cut-outs (106), wherein the cut-outs (106) each have a length less than 10% of the wavelength in free-space at the operating frequency.

2. A microwave ablation system according to claim 1, wherein the plurality of cut-outs (106) each have a length less than half a wavelength at the operating frequency in the medium within the cut-outs (106).

3. A microwave ablation system according to claim 1 or claim 2, wherein the plurality of cut-outs (106) each have a length within a range of between 1 mm to 8 mm, or a length less than 4 mm.

4. A microwave ablation system according to any preceding claim, wherein the plurality of cut-outs (106) each extend along a helical path around the outer conductor (202c), such that each cut-out extends around a longitudinal axis of the coaxial cable (102), and optionally:
wherein each of the plurality of cut-outs (106) extend along part of a combined continuous helical path along the length of the coaxial cable (102), and are spaced apart along the continuous helical path to form an intermittent helical cut-pattern (602).

5. A microwave ablation system according to any of claims 1 to 3, wherein the plurality of cut-outs (106) each extend only partly around the outer conductor (202c) around an axis of the coaxial cable (102), preferably in a plane normal to the longitudinal axis, each spaced by a distance along the length of the coaxial cable (102).

6. A microwave ablation system according to claim 5, wherein each of the plurality of cut-outs (106) are offset around the longitudinal axis of the coaxial cable (102) relative to an adjacent cut-out, and optionally one or both of:
a) the plurality of cut-outs (106) are grouped together, wherein the groups are spaced apart along the length of the coaxial cable (102) and each consecutive cut-out in a group is offset in relation to an adjacent cut-out in the group of cut-outs; and/or
b) each of the plurality of cut-outs (106) is offset relative to adjacent cut-outs along the length of the coaxial cable (102) such that the plurality of cut-outs (106) are arranged in a brick pattern (402).

7. A microwave ablation system according to any of the preceding claims, wherein the plurality of cut-outs (106) each extend completely through a thickness of the outer conductor (202c),
and preferably wherein the plurality of cut-outs (106) each only extend through the outer conductor (202c), and do not extend into the dielectric (202b).

8. A microwave ablation system according to any preceding claim, wherein the operating frequency is in a range between 10.0 GHz to 0.5 GHz, or is preferably in a range between 2.4GHz to 2.5GHz, and is further preferably 2.45GHz.

9. A microwave ablation system according to any preceding claim, wherein the plurality of cut-outs (106) is arranged to form a first portion (702) of the coaxial cable (102) having a first degree of flexibility and a second portion (704) of the coaxial cable (102) having a second degree of flexibility greater than the first, and optionally wherein:
the plurality of cut-outs (106) in the first portion (702) has a lower number density compared to those of the second portion (704).

10. A microwave ablation system according to any preceding claim, wherein the feeding cable (102) further comprises a sheath (114) around the coaxial cable (102).

11. A microwave ablation system according to claim 10 wherein the sheath (114) is arranged to form a water-resistant barrier around the coaxial cable (102), and optionally wherein the sheath (102) is formed from: a dip coating of the coaxial cable (102), a shrink-fit polymer tube around the coaxial cable (102), or an extruded jacket over the coaxial cable (102).

12. A microwave ablation system according to claim 10 or claim 11, wherein the sheath (114) comprises a metalized layer around the coaxial cable (102).

13. A microwave ablation system according to claim 10 or claim 11 or claim 12, wherein the feeding cable (101) further comprises one or more conducting wires (116) arranged between the outer surface of the coaxial cable (102) and the sheath (114), the conducting wires (116) extending along the length of the coaxial cable (102) and electrically connecting at least some of the plurality of cut-outs (106), and optionally wherein the one or more conducting wires (116) may form a conducting braid or coil around the feeding cable (101).

14. A method of making the coaxial cable of the microwave ablation system of any preceding claim, the method comprising:
providing (1001) a coaxial cable (102) having: an inner conductor (202a), an outer conductor (202c); and a dielectric (202b) arranged between the inner conductor (202a) and the outer conductor (202c); and
cutting (1002) a plurality of cut-outs (106) in the outer conductor (202c) using a laser beam to remove material from the outer conductor (202c), wherein the cut-outs (106) each have a length less than 10% of the wavelength in free-space at the operating frequency of the microwave ablation system.

15. A method according to claim 14, wherein cutting the plurality of cuts-outs (106) comprises controlling the laser beam by tuning the laser beam to remove material from the outer conductor (202c) but not from the dielectric (202b).

## Patentansprüche

1. Mikrowellenablationssystem (100), Folgendes umfassend:
einen Signalgenerator (108), der eingerichtet ist, um ein Signal auf einer Betriebsfrequenz zu generieren, bei der Mikrowellenenergie an ein Zielgewebe abgegeben wird;
eine Mikrowellenablationssonde (104), die einen Applikator (112) umfasst, der eingerichtet ist, um Mikrowellenstrahlung auf einen Zielort zu emittieren; und
ein Speisekabel (101), das eingerichtet ist, um den Applikator (112) mit dem Signalgenerator (108) zu verbinden, wobei das Speisekabel (101) ein Koaxialkabel (102) umfasst, das zwischen dem Signalgenerator (108) und dem Applikator (112) eingebunden ist, wobei das Koaxialkabel (102) Folgendes umfasst:
einen Innenleiter (202a);
einen Außenleiter (202c) und
ein Dielektrikum (202b), das zwischen dem Innenleiter (202a) und dem Außenleiter (202c) angeordnet ist,
wobei der Außenleiter (202c) eine Vielzahl von Aussparungen (106) umfasst, wobei die Aussparungen (106) jeweils eine Länge von weniger als 10 % der Wellenlänge im Freiraum bei der Betriebsfrequenz aufweisen.

2. Mikrowellenablationssystem nach Anspruch 1, wobei die Vielzahl von Aussparungen (106) jeweils eine Länge von weniger als einer halben Wellenlänge bei der Betriebsfrequenz in dem Medium in den Aussparungen (106) aufweisen.

3. Mikrowellenablationssystem nach Anspruch 1 oder 2, wobei die Vielzahl von Aussparungen (106) jeweils eine Länge im Bereich zwischen 1 mm und 8 mm oder eine Länge von weniger als 4 mm aufweisen.

4. Mikrowellenablationssystem nach einem vorhergehenden Anspruch, wobei sich die Vielzahl von Aussparungen (106) jeweils entlang eines spiralförmigen Weges rings um den Außenleiter (202c) erstrecken, so dass sich jede Aussparung rings um eine Längsachse des Koaxialkabels (102) erstreckt, und optional
wobei sich optional die mehreren Aussparungen (106) jeweils über einen Teil eines kombinierten durchgehenden spiralförmigen Weges über die Länge des Koaxialkabels (102) erstrecken und entlang des durchgehenden spiralförmigen Weges beabstandet sind, um ein unterbrochenes spiralförmiges Aussparungsmuster (602) zu bilden.

5. Mikrowellenablationssystem nach einem der Ansprüche 1 bis 3, wobei sich die Vielzahl von Aussparungen (106) jeweils nur teilweise rings um den Außenleiter (202c) rings um eine Achse des Koaxialkabels (102) erstrecken, vorzugsweise in einer Ebene, die senkrecht zu der Längsachse liegt, jeweils in einem Abstand über die Länge des Koaxialkabels (102).

6. Mikrowellenablationssystem nach Anspruch 5, wobei die Vielzahl von Aussparungen (106) jeweils rings um die Längsachse des Koaxialkabels (102) relativ zu einer benachbarten Aussparung versetzt sind und optional eines oder beides des Folgenden gilt:
a) die Vielzahl von Aussparungen (106) sind miteinander gruppiert, wobei die Gruppen über die Länge des Koaxialkabels (102) beabstandet sind und jede fortlaufende Aussparung in einer Gruppe in Bezug auf eine benachbarte Aussparung in der Gruppe von Aussparungen versetzt ist; und/oder
b) jede der Vielzahl von Aussparungen (106) relativ zu benachbarten Aussparungen über die Länge des Koaxialkabels (102) derart versetzt ist, dass die Vielzahl von Aussparungen (106) in einem Ziegelmuster (402) angeordnet sind.

7. Mikrowellenablationssystem nach einem der vorhergehenden Ansprüche, wobei sich die Vielzahl von Aussparungen (106) jeweils vollständig durch eine Dicke des Außenleiters (202c) erstrecken,
und wobei sich vorzugsweise die Vielzahl von Aussparungen (106) jeweils nur durch den Außenleiter (202c) erstrecken und sich nicht in das Dielektrikum (202b) erstrecken.

8. Mikrowellenablationssystem nach einem vorhergehenden Anspruch, wobei die Betriebsfrequenz in einem Bereich zwischen 10,0 GHz und 0,5 GHz liegt oder vorzugsweise in einem Bereich zwischen 2,4 GHz und 2,5 GHz liegt und ferner vorzugsweise 2,45 GHz beträgt.

9. Mikrowellenablationssystem nach einem vorhergehenden Anspruch, wobei die Vielzahl von Aussparungen (106) eingerichtet sind, um einen ersten Abschnitt (702) des Koaxialkabels (102) zu bilden, der einen ersten Flexibilitätsgrad aufweist, und einen zweiten Abschnitt (704) des Koaxialkabels (102), der einen zweiten Flexibilitätsgrad aufweist, der größer als der erste ist, und wobei optional:
die Vielzahl von Aussparungen (106) in dem ersten Abschnitt (702) eine Dichte geringerer Anzahl im Vergleich mit denen des zweiten Abschnitts (704) aufweisen.

10. Mikrowellenablationssystem nach einem vorhergehenden Anspruch, wobei das Speisekabel (102) ferner eine Hülle (114) rings um das Koaxialkabel (102) umfasst.

11. Mikrowellenablationssystem nach Anspruch 10, wobei die Hülle (114) eingerichtet ist, um eine wasserfeste Sperre rings um das Koaxialkabel (102) zu bilden, und wobei optional die Hülle (102) aus Folgendem gebildet ist: einer Tauchbeschichtung des Koaxialkabels (102), einem Polymerschrumpfschlauch rings um das Koaxialkabel (102) oder einem über das Koaxialkabel (102) extrudierten Mantel.

12. Mikrowellenablationssystem nach Anspruch 10 oder Anspruch 11, wobei die Hülle (114) eine metallisierte Schicht rings um das Koaxialkabel (102) umfasst.

13. Mikrowellenablationssystem nach Anspruch 10 oder Anspruch 11 oder Anspruch 12, wobei das Speisekabel (101) ferner einen oder mehrere leitende Drähte (116) umfasst, die zwischen der Außenfläche des Koaxialkabels (102) und der Hülle (114) angeordnet sind, wobei sich die leitenden Drähte (116) über die Länge des Koaxialkabels (102) erstrecken und mindestens einige der Vielzahl von Aussparungen (106) elektrisch verbinden, und wobei optional der eine oder die mehren leitenden Drähte (116) ein leitendes Geflecht oder eine leitende Spule rings um das Speisekabel (101) bilden können.

14. Verfahren zur Herstellung des Koaxialkabels des Mikrowellenablationssystems nach einem vorhergehenden Anspruch, wobei das Verfahren Folgendes umfasst:
Bereitstellen (1001) eines Koaxialkabels (102), das Folgendes aufweist: einen Innenleiter (202a), einen Außenleiter (202c) und ein Dielektrikum (202b), das zwischen dem Innenleiter (202a) und dem Außenleiter (202c) angeordnet ist; und
Schneiden (1002) einer Vielzahl von Aussparungen (106) in den Außenleiter (202c) mit Hilfe eines Laserstrahls, um Material aus dem Außenleiter (202c) zu entfernen, wobei die Aussparungen (106) jeweils eine Länge von weniger als 10 % der Wellenlänge im Freiraum bei der Betriebsfrequenz des Mikrowellenablationssystems aufweisen.

15. Verfahren nach Anspruch 14, wobei das Schneiden der Vielzahl von Aussparungen (106) das Steuern des Laserstrahls durch Abstimmen des Laserstrahls, um Material von dem Außenleiter (202c), jedoch nicht von dem Dielektrikum (202b) zu entfernen, umfasst.

## Revendications

1. Système d'ablation par micro-ondes (100), comprenant :
un générateur de signal (108), agencé de manière à générer un signal à une fréquence de fonctionnement à laquelle une énergie micro-ondes est délivrée à un tissu cible ;
une sonde d'ablation par micro-ondes (104) comprenant un applicateur (112) agencé de manière à émettre un rayonnement micro-ondes vers un emplacement cible ; et
un câble d'alimentation (101) agencé de manière à connecter l'applicateur (112) au générateur de signal (108), le câble d'alimentation (101) comprenant un câble coaxial (102) connecté entre le générateur de signal (108) et l'applicateur (112), le câble coaxial (102) comprenant :
un conducteur interne (202a) ;
un conducteur externe (202c) ; et
un diélectrique (202b) agencé entre le conducteur interne (202a) et le conducteur externe (202c),
dans lequel le conducteur externe (202c) comprend une pluralité de découpes (106), les découpes (106) présentant chacune une longueur inférieure à 10 % de la longueur d'onde dans l'espace libre à la fréquence de fonctionnement.

2. Système d'ablation par micro-ondes selon la revendication 1, dans lequel la pluralité de découpes (106) présentent chacune une longueur inférieure à une demi-longueur d'onde à la fréquence de fonctionnement dans le milieu présent au sein des découpes (106).

3. Système d'ablation par micro-ondes selon la revendication 1 ou la revendication 2, dans lequel la pluralité de découpes (106) présentent chacune une longueur comprise dans une plage allant de 1 mm à 8 mm, ou une longueur inférieure à 4 mm.

4. Système d'ablation par micro-ondes selon l'une quelconque des revendications précédentes, dans lequel la pluralité de découpes (106) s'étendent chacune le long d'une trajectoire hélicoïdale autour du conducteur externe (202c), de sorte que chaque découpe s'étend autour d'un axe longitudinal du câble coaxial (102), et facultativement :
la pluralité de découpes (106) s'étendent chacune le long d'une partie d'une trajectoire hélicoïdale continue combinée sur la longueur du câble coaxial (102), et sont espacées le long de la trajectoire hélicoïdale continue pour former un motif de découpes hélicoïdal intermittent (602).

5. Système d'ablation par micro-ondes selon l'une quelconque des revendications 1 à 3, dans lequel la pluralité de découpes (106) s'étendent chacune uniquement partiellement autour du conducteur externe (202c) autour d'un axe du câble coaxial (102), de préférence dans un plan normal à l'axe longitudinal, chacune étant espacée d'une certaine distance sur la longueur du câble coaxial (102).

6. Système d'ablation par micro-ondes selon la revendication 5, dans lequel chacune de la pluralité de découpes (106) est décalée autour de l'axe longitudinal du câble coaxial (102) par rapport à une découpe adjacente, et facultativement :
a) la pluralité de découpes (106) sont regroupées, les groupes étant espacés les uns des autres sur la longueur du câble coaxial (102) et chaque découpe consécutive dans un groupe étant décalée par rapport à une découpe adjacente dans le groupe de découpes ; et/ou
b) chacune de la pluralité de découpes (106) est décalée par rapport à des découpes adjacentes sur la longueur du câble coaxial (102) de sorte que la pluralité des découpes (106) sont disposées selon un motif en briques (402).

7. Système d'ablation par micro-ondes selon l'une quelconque des revendications précédentes, dans lequel la pluralité de découpes (106) s'étendent chacune complètement à travers une épaisseur du conducteur externe (202c),
et, de préférence, dans lequel la pluralité de découpes (106) s'étendent chacune uniquement à travers le conducteur externe (202c) sans s'étendre jusque dans le diélectrique (202b).

8. Système d'ablation par micro-ondes selon l'une quelconque des revendications précédentes, dans lequel la fréquence de fonctionnement est comprise dans une plage allant de 0,5 GHz à 10,0 GHz, ou est de préférence comprise dans une plage allant de 2,4 GHz à 2,5 GHz, et est de préférence encore de 2,45 GHz.

9. Système d'ablation par micro-ondes selon l'une quelconque des revendications précédentes, dans lequel la pluralité de découpes (106) sont agencées pour former un premier tronçon (702) du câble coaxial (102) présentant un premier degré de souplesse et un deuxième tronçon (704) du câble coaxial (102) présentant un deuxième degré de souplesse supérieur au premier, et facultativement dans lequel :
la pluralité de découpes (106) dans le premier tronçon (702) présentent une densité en nombre inférieure à celle des découpes du deuxième tronçon (704).

10. Système d'ablation par micro-ondes selon l'une quelconque des revendications précédentes, dans lequel le câble d'alimentation (102) comprend en outre une gaine (114) autour du câble coaxial (102).

11. Système d'ablation par micro-ondes selon la revendication 10, dans lequel la gaine (114) est agencée de manière à former une barrière résistante à l'eau autour du câble coaxial (102), et facultativement dans lequel la gaine (102) est formée à partir de : un revêtement au trempé du câble coaxial (102), un tube polymère thermorétractable autour du câble coaxial (102), ou une enveloppe extrudée par-dessus le câble coaxial (102).

12. Système d'ablation par micro-ondes selon la revendication 10 ou la revendication 11, dans lequel la gaine (114) comprend une couche métallisée autour du câble coaxial (102).

13. Système d'ablation par micro-ondes selon la revendication 10 ou la revendication 11 ou la revendication 12, dans lequel le câble d'alimentation (101) comprend en outre un ou plusieurs fils conducteurs (116) agencés entre la surface externe du câble coaxial (102) et la gaine (114), les fils conducteurs (116) s'étendant le long du câble coaxial (102) et connectant électriquement au moins certaines de la pluralité de découpes (106), et facultativement dans lequel le ou les fils conducteurs (116) peuvent former une tresse conductrice ou un enroulement conducteur autour du câble d'alimentation (101).

14. Procédé de fabrication du câble coaxial du système d'ablation par micro-ondes selon l'une quelconque des revendications précédentes, le procédé comprenant :
la fourniture (1001) d'un câble coaxial (102) présentant : un conducteur interne (202a), un conducteur externe (202c) ; et un diélectrique (202b) agencé entre le conducteur interne (202a) et le conducteur externe (202c) ; et
la découpe (1002) d'une pluralité de découpes (106) dans le conducteur externe (202c) au moyen d'un faisceau laser afin d'enlever de la matière du conducteur externe (202c), les découpes (106) présentant chacune une longueur inférieure à 10 % de la longueur d'onde dans l'espace libre à la fréquence de fonctionnement du système d'ablation par micro-ondes.

15. Procédé selon la revendication 14, dans lequel la découpe de la pluralité de découpes (106) comprend la commande du faisceau laser par réglage du faisceau laser afin d'enlever de la matière du conducteur externe (202c) sans enlever de matière du diélectrique (202b).
